# EUROPEAN PATENT SPECIFICATION

(11) **EP 3 411 014 B1**
(45) Date of publication and mention of the grant of the patent: **14.04.2021**
(21) Application number: 17708583.4
(22) Date of filing: 02.02.2017
(51) Int. Cl.: A61K 9/06, A61P 17/00

(54) **NEW TOPICAL COMPOSITIONS COMPRISING USNIC ACID AND THEIR USE IN THERAPY**
NEUE TOPISCHE ZUSAMMENSETZUNGEN MIT USNINSÄURE UND DEREN THERAPEUTISCHE VERWENDUNG
NOUVELLES COMPOSITIONS TOPIQUES COMPRENANT DE L'ACIDE USNIQUE ET LEUR UTILISATION THÉRAPEUTIQUE

(30) Priority: 04.02.2016 IT UB20160024
(43) Date of publication of application: 12.12.2018
(73) Proprietor: Apharm S.r.l., 28041 Arona (NO) (IT)
(72) Inventor: PIZZONI, Angelo, 28041 Arona (NO) (IT); PIZZONI, Paolo, 28041 Arona (NO) (IT)
(74) Representative: Trupiano, Federica
(86) International application number: PCT/IB2017/050566
(87) International publication number: WO 2017/134594

(56) References cited:
- EP-A1- 1 374 903
- WO-A1-2012/131347
- WO-A2-2012/049677

## Description

### Background of the Invention

The present invention refers to new topical compositions comprising usnic acid. The invention also comprises the use of said compositions, possibly in association with other active principles, for the therapy of skin diseases, including dermatitis, eczemas, skin wounds of various origin, sores, burns, psoriasis, proliferative keratosis and skin diseases caused by microbial and fungal agents.

### Technical field

Many skin diseases are known. Psoriasis, which is a widespread skin proliferative keratosis appearing with red spots and wounds covered by whitish scales, is one of the most disabling proliferative keratosis. The wounds are particularly localized in the knees, sacral region, elbows, hands, feet and scalp. Psoriasis is a chronic disease, characterized by periods of remission alternating phases of exacerbation.

Many other skin diseases are caused by microbial and fungal agents, such as yeasts and fungi.

Usnic acid, or 2,6-diacetyl-7,9-dihydroxy-8,9b-dimethyl-1,3(2H,9bh)-dibenzo furandione, having structural formula (I) 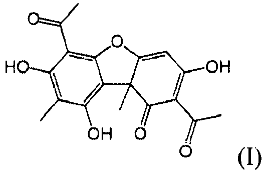 is a known compound, originally extracted from some species of lichens, showing anti-microbial and healing properties. The use of usnic acid is also known in the therapy of skin proliferative diseases such as psoriasis.

It is also known the poor water-solubility of usnic acid, that makes it difficult to prepare water-based compositions of usnic acid, especially at high concentration. In order to avoid this drawback, usnic acid is often solubilized with irritant organic solvents, for example ethanol, methanol and acetone, for its use in pharmaceutical and cosmetic compositions. However, the presence of irritant organic solvents is not acceptable in the topical compositions and is forbidden for this reason.

WO 2012 / 131 347 A1 discloses a formulation for the topical antimicrobial treatment of skin comprising usnic acid or a salt thereof, dissolved in a solvent system comprising dimethyl isosorbide, a C1 to C9 alkyl salicylate and a glyceryl fatty acid ester, which assists in the effective dissolution of the usnic acid or usnate.

Therefore there is the need to find water-based, topical, pharmaceutical or cosmetic compositions comprising high concentration of usnic acid but free of organic solvents that are irritant for the cutis.

### Objects of the Invention

An object of the invention is to provide topical compositions containing usnic acid in high concentrations and the use thereof for the treatment and prevention of skin diseases.

An object of the invention is to provide the compositions above, in association with other active principles.

Another object of the invention is to provide a method for the treatment of skin diseases, comprising applying the compositions of the invention on the affected areas.

Another object of the invention is to provide a mixture of solubilizing agents able to satisfactorily solubilize and stabilize the usnic acid.

### Description of the invention

According to one of its aspects, subject-matter of the invention is a topical composition comprising usnic acid, or a salt thereof, and a solubilizer consisting of a mixture of at least one ethoxylated hydrogenated castor oil derivative and at least one polypropylene glycol buteth.

According to a preferred embodiment, the composition of the invention does not contain solvents irritant for the skin, such as ethanol, methanol and acetone.

Usnic acid has the structural formula (I) depicted above. According to the present invention usnic acid in unsalified form is preferred.

By "salts" herein is always meant the pharmaceutically acceptable salts.

According to a preferred embodiment, the ethoxylated hydrogenated castor oil derivative is a PEG hydrogenated castor oil, advantageously PEG-40 hydrogenated castor oil.

According to another preferred embodiment, the polypropylene glycol buteth is polypropylene glycol-26-buteth-26 (also called "PPG-26-buteth-26).

According to another particularly preferred embodiment, said solubilizer consists of PEG-40 hydrogenated castor oil and polypropylene glycol-26-buteth-26, advantageously in a PEG-40 hydrogenated castor oil + polypropylene glycol-26-buteth-26 weight ratio from 0.5/1 to 1.5/1, for example of about 0.8/1.

According to a preferred embodiment, the weight ratio of unsalified usnic acid and said solubilizer is from 1/1 to 1/2, advantageously from 1/1 to 1/1.3-1.5.

The presence of said solubilizer allows obtaining water-based topical compositions for example, but not only, in the form of emulsions, comprising up to 4% usnic acid, advantageously up to 3% usnic acid, in fact said solubilizer provides clear solutions that can be added to the remainder of the water-based composition that is kept stable over time. This result is particularly interesting, because it allows formulating topical compositions, in particular water-based emulsions, in the absence of solvents that are irritant for the skin such as, for example, ethanol, methanol and acetone.

According to a preferred embodiment, the composition of the invention comprises hyaluronic acid or a salt, advantageously sodium hyaluronate. According to the invention a hyaluronic acid can be employed, preferably in the form of sodium salt, of any molecular weight or also mixtures of different molecular weight.

According to an advantageous embodiment, the hyaluronic acid (or a salt thereof, advantageously sodium salt) has low average molecular weight, advantageously from 50,000 to 300,000 Da, preferably from 80,000 to 120,000 Da, more preferably about 100,000 Da.

According to the present invention, the hyaluronic acid (or the salts thereof, preferably the sodium salt thereof) is meant in a non-crosslinked or crosslinked form. Preferably, the hyaluronic acid and the salts thereof, advantageously its sodium salt, are in the non-crosslinked form. However, it is also possible to use hyaluronic acid and the salts thereof, preferably its sodium salt, in crosslinked form with conventional crosslinking agents, by way of example with a crosslinking agent selected from 1,4-butanediol diglycidyl ether (BDDE), divinylsulphone (DVS) and polyethylene glycol (PEG).

In a preferred composition according to the invention, usnic acid and sodium hyaluronate are in a weight ratio from 10/1 to 1/1 (w/w) respectively, preferably from 5/1 to 1/1 (w/w), more preferably in usnic acid/sodium hyaluronate weight ratios around 2.5/1. However different ratios can be used and are comprised within the scope of protection of the invention.

The composition of the invention proved to be very effective in the treatment of many skin diseases, including eczemas, dermatitis, skin wounds of various origin, sores, burns, psoriasis and proliferative keratosis.

Also other skin diseases can be treated with the composition of the invention as well, in particular, being usnic acid an anti-microbial, all of the diseases caused by microbial and fungal agents, dandruff included.

The composition of the invention can also comprise other pharmaceutically acceptable active principles and/or excipients.

According to an embodiment, the compositions of the invention also comprise anti-fungal agents, in particular ketoconazole or ciclopirox, said compositions being useful for the treatment of dermatitis, among which seborrheic dermatitis, and all of the resulting symptomatology, such as for example itch, dandruff, greasiness etc. The anti-fungal agents, preferably ketoconazole, are advantageously added at a rate of 1.5-3%, for example 1%, 1.5% or 2%, by weight, relative to the total weight of the composition.

Possibly, other active principles can be added to the composition of the invention, such as for example anti-inflammatory agents.

Naturally occurring anti-inflammatory agents, such as for example Indica naturalis; tumeric acid; aloe vera; capsaicin; isoflavonoids such as quercetin and hesperidin; salicin; tea tree oil; yarrow (from achillea flowers); and essential fatty acids, such as gamma-linolenic acid (GLA) contained in borage and primrose.

Other anti-inflammatory agents that can be used are for example non-steroidal anti-inflammatory agents, such as for example diclofenac and salicylic acid, or steroidal anti-inflammatory agents such as clobetasol, hydrocortisone, dexamethasone and betamethasone.

Other active principles that can be added to the mixture are for example selenium sulfide, pynthione, resorcinol, podophyl, podofilox, anthralin, methoxsalen.

Further active principles that can be added to the mixture are vitamin derivatives, such as vitamin D and vitamin A derivatives, for example calcipotriene, calcitriol and tazarotene.

In a clinical assay, whose protocol is quoted in the experimental section of the present description, a representative composition of the invention showed particular effectiveness in the treatment of psoriasis, by significantly reducing wounds and preventing the formation of new plaques.

The use of the composition of the invention in the treatment of the diseases mentioned above constitutes a further aspect of the present invention.

For its administration, the composition preferably comprises excipients and vehicles suitable for the topical application. Such compositions can be, for example, in the form of cream (oil in water or water in oil), emulsion, gel, solution, oil, powder, ointments, pastes, foams and sprays. Shampoos for cleansing the scalp can also be prepared for example, but not only, when also an anti-fungal agent is used, such as also foams for easy and quick applications. Alternatively, said compositions can be formulated in combination with a patch and thus be in the form of patch to be applied on the skin. The composition can be packaged in mono- or multi-dose containers suitable for the topical application, for example in tubes, bottles, pots, etc. According to a preferred embodiment, the compositions of the invention can also comprise isopropyl myristate; in fact it has been observed that this compound contributes to convey usnic acid and further offers flowability and softness to the composition that proves to be more homogeneous and fluid.

Thus, preferred compositions comprise usnic acid, the above defined solubilizing mixture, hyaluronic acid or one of the salts thereof and isopropyl myristate.

Preferred compositions comprise from 0.5 to 10%, advantageously from 1 to 5%, more preferably about 3 wt% of usnic acid or a salt thereof, preferably usnic acid, from 0.5 to 5%, advantageously from 1 to 3%, more preferably about 1.2-1.3 wt% of hyaluronic acid or a salt thereof, preferably sodium hyaluronate, said amounts being expressed by weight relative to the total weight of the composition.

When isopropyl myristate is present in the composition, it is present at a rate of 0.5-10%, advantageously 1-5%, more preferably about 3 wt%, said amounts being expressed by weight relative to the total weight of the composition.

The compositions of the invention also advantageously comprise pharmaceutically acceptable conventional excipients and vehicles. One skilled in the art is perfectly able to select such vehicles and excipients, depending on the type of composition to be prepared.

By way of example, the compositions can contain water, solubilizing agents (in addition to the above mentioned solubilizing mixture), emulsifiers, surfactants, preservatives, moisturizers, antioxidants, gelling agents, chelating, antioxidants, emollients, humectants, anti-statics, detergents, rheological additives, lipophilic substances, colorants and perfumes.

Starting from the above described composition of the invention, wherein the usnic acid is solubilized and stabilized by the mixture of at least one ethoxylated hydrogenated castor oil derivative and at least one polypropylene glycol buteth as defined above, advantageously but not necessarily, in association with hyaluronic acid or a salt thereof and/or isopropyl myristate, one skilled in the art is perfectly able to formulate a topical composition for use in the above mentioned diseases. However preferred compositions are quoted in the experimental section of the present description, by way of illustration and in no way limitative.

According to another of its aspects, subject-matter of the invention is a method for the treatment of skin diseases, including eczemas, dermatitis, psoriasis, proliferative keratosis and skin diseases caused by microbial agents, comprising administering by topical route the composition of the invention, to a subject in the need thereof.

The composition of the invention can be administered once or more times per day depending on the type of disease and the subject to be treated.

Being free of irritants, the composition can also be used on the injured cutis in particular, but not only, when it also comprises hyaluronic acid, advantageously in the form of the sodium salt thereof.

According to another of its aspects, subject-matter of the invention is the use of a mixture of at least one ethoxylated hydrogenated castor oil derivative and at least one polypropylene glycol buteth, advantageously the preferred mixtures as indicated above, to solubilize and stabilize usnic acid in water-based compositions, advantageously free of irritant solvents such as, for example, ethanol, methanol and acetone.

### Experimental Section

### Example 1

An emulsion comprising 3% usnic acid having the following formula is prepared

| **INCI NAME** | **AMOUNT (grams)** |
|---|---|
| AQUA | Qs to 1,000.00 |
| PARAFFINUM LIQUIDUM | 60.00 |
| CETEARYL ALCOHOL | 45.00 |
| GLYCERYL STEARATE | 35.00 |
| ISOPROPYL MYRISTATE | 30.00 |
| USNIC ACID | 30.00 |
| PROPYLENE GLYCOL | 30.00 |
| CETYL PALMITATE | 25.00 |
| PPG-26-BUTETH-26 | 20.00 |
| PEG-40 HYDROGENATED CASTOR OIL | 16.00 |
| POTASSIUM PALMITOYL HYDROLYZED WHEAT PROTEIN | 15.00 |
| NON-CROSSLINKED SODIUM HYALURONATE | 12.49 |
| CAPRYLIC/CAPRIC TRIGLYCERIDE | 9.90 |
| POLYSORBATE-80 | 6.64 |
| PHENOXYETHANOL | 6.00 |
| PEG-12 | 5.00 |
| CARBOMER | 3.00 |
| METHYLPARABEN | 1.20 |
| CETYL ACETATE | 1.04 |
| ETHYLPARABEN | 0.80 |
| SODIUM HYDROXIDE | 0.60 |
| TETRASODIUM EDTA | 0.50 |
| ACETYLATED LANOLIN ALCOHOL | 0.16 |
| BHT | 0.02 |
| PROPYL GALLATE | 0.02 |

### Example 2

A topical emulsion of example 1 is prepared with the following process:

| PHASE | INCI |
|---|---|
| A | ACETYLATEDLANOL IN AL COHOL+CETYL ACETATE+POLYSORBATE-80 |
| A | CETYL PALMITATE |
| A | BHT+CAPRYLIC/CAPRIC TRTGLYCERIDE+PROPYL GALLATE |
| A | CETEARYL ALCOHOL |
| A | PARAFFINUM LIQUIDUM |
| B | AQUA |
| R | TETRASODIUM EDTA |
| R | PROPYLENE GLYCOL |
| C | CARBOMER |
| r | SODIUM HYALURONATE |
| D | USNIC ACID |
| D | PEG-40 HYDROGENATED CASTOR OIL+PPG-26-BUTETH-26 |
| D | ISOPROPYL MYRISTATE |
| E | ETHYL PARABEN+METHYLPARA BEN + PHENOXYETHANOL |
| F | ACQUA+SODIUM HYDROXIDE |

Putting the components of phase A together and heating to 80°C. Putting the components of phase B together and heating to 80°C. Emulsifying the two phases, under a turboemulsifier for 10 minutes at 4000 rpm. Adding the components of phase C hot, stirring until perfect dispersion of the powders. Preparation of phase D: solubilizing usnic acid in the solubilizing mixture of phase D. When the mixture is perfectly clear, putting the phase D hot, together with the previously prepared emulsion. Stirring with turboemulsifier for 5 minutes at 4000 rpm. Cooling the emulsion down to 30°C and adding the E and F components to it. Packing in suitable containers.

### Example 3

An emulsion comprising 3% usnic acid having the following formula is prepared

| **INCI NAME** | **AMOUNT (grams)** |
|---|---|
| AQUA | Qs to 1,000.00 |
| PARAFFINUM LIQUIDUM | 60.00 |
| CETEARYL ALCOHOL | 45.00 |
| GLYCERYL STEARATE | 35.00 |
| ISOPROPYL MYRISTATE | 30.00 |
| USNIC ACID | 30.00 |
| PROPYLENE GLYCOL | 30.00 |
| CETYL PALMITATE | 25.00 |
| PPG-26-BUTETH-26 | 20.00 |
| PEG-40 HYDROGENATED CASTOR OIL | 16.00 |
| POTASSIUM PALMITOYL HYDROLYZED WHEAT PROTEIN | 15.00 |
| NON-CROSSLINKED SODIUM HYALURONATE | 12.49 |
| KETOCONAZOLE | 15.00 |
| CAPRYLIC/CAPRIC TRIGLYCERIDE | 9.90 |
| POLYSORBATE-80 | 6.64 |
| PHENOXYETHANOL | 6.00 |
| PEG-12 | 5.00 |
| CARBOMER | 3.00 |
| METHYLPARABEN | 1.20 |
| CETYL ACETATE | 1.04 |
| ETHYLPARABEN | 0.80 |
| SODIUM HYDROXIDE | 0.60 |
| TETRASODIUM EDTA | 0.50 |
| ACETYLATED LANOLIN ALCOHOL | 0.16 |
| BHT | 0.02 |
| PROPYL GALLATE | 0.02 |

### Example 4

### Clinical Study

The purpose of the study was to determine the effectiveness, systemic safety, local tolerability of the topical formulation of Example 1 on a mild to moderate plaque psoriasis.

### Clinical Protocol

Twenty-one patients have been included in an open study and treated twice per day with the emulsion of example 1 for two weeks, in a single selected area containing a target plaque. In the days of the visit during the study, it was allowed to wash themselves but not to hydrate before participating. At the end of the study and after the final visit, the area to be treated has been left without treatment for 10 days of follow-up. 3 visits have been performed: at the baseline (Visit 1), after 2 weeks of treatment (Visit 2) and 10 days after the last administration (Visit 3).

### Inclusion Criteria

▪ Age ≥ 18 and ≤ 60 years old
▪ Both sexes. Non-pregnant women accepted to use a suitable contraceptive method during the study.
▪ Diagnosis performed by a dermatologist, of a stable, chronic, plaque psoriasis, from at least 6 months prior to the first application.
▪ Presence of mild to moderate plaque psoriasis covering ≤ 10% of the total Body Surface Area (BSA), "Psoriasis Area Severity Index" (PASI) <20 and a Total Plaque Area to be treated (TPA) ≥ 9 cm2 and ≤ 100 cm2 with "Target Plaque Severity Score" (TSS) ≥ 5 and "induration subscore" ≥ 2

### Exclusion Criteria

▪ Forms of non-plaque (erythrodermic, guttate, pustular) psoriasis, drug-induced psoriasis (for example beta blockers, Ca-antagonists, lithium), history of psoriatic arthritis or recent systemic or local infection. Concomitant dermatological disorders.
▪ Hypersensitivity to sunlight, any disease known to be aggravated by UV exposure.
▪ Pregnancy, breastfeeding.
▪ Systemic treatments that could have influenced psoriasis, such as oral or injectable corticosteroids, retinoids, methotrexate and cyclosporine in the 4 weeks prior to the first application.
▪ Topical drugs for the treatment of plaque psoriasis (steroids of the OMS I-II group, vitamin D derivatives, tar, keratolytics) have been interrupted for ≥ 2 weeks prior to the first application of the emulsion of the study. As an exception, hydrocortisone ≤ 1% (for the use on palms, soles of the feet, face and intertriginous area, for the scalp only preparations with tar or salicylic acid without corticosteroids) has been allowed.
▪ Use of PUVA or phototherapy in the previous 3 months.
▪ Use of biological or conventional systemic agents for the treatment of psoriasis in the previous 6 months.

### Rating

Rating was made on:
▪ Absolute value and percentage variation relative to the baseline in the TSS of the target plaque on week 2 (sum of erythema, desquamation, "elevation scores").
▪ Variation relative to the baseline in the different TSS scores: the rating of reliefs, resizing and erythema with a scale of severity of 5 points (0 = none, 1 = mild, 2 = moderate, 3 = marked, 4 = very marked). The partial scores have been rated at the baseline and at the visit of the second week.
▪ Variation of the area of destination of the plaque (TPA) on the second week. The perimeter of the plaque has been marked out at the baseline and at the visit of the second week.
▪ Variation of the "Itch Severity Index" (ISI) relative to the baseline, measured in the 24 hours prior to the visit (0 = no itch, 1 = mild itch, 2 = moderate itch, 3 = strong itch) at the baseline and at the visit of the second week.
▪ Physician global assessment (PGA) on a scale of 6 points (1 = severe, 2 = moderate to severe, 3 = moderate, 2 = mild, 1 = almost healed, 0 = healed) rated at the baseline and at the visit of the second week.
▪ Patient global assessment of tolerability of the new agent, cosmetic acceptability, ease of use (1 = good, 2 = moderate, 3 = poor, 4 = not acceptable) at the visit of the second week.
▪ Safety and tolerability: incidence and severity of the systemic adverse events, local tolerability rated on the plaque area at the visit of the second week and at the follow-up visit.
▪ Number of relapses defined as ≥ 50% loss of "gain" and number of "rebounds" defined as the worsening of psoriasis relative to the baseline value or new pustular, erythrodermic psoriasis or more inflammation that would verify within 10 days after stopping the therapy.
▪ Duration of the remission (complete healing), durability of the response in case of significant improvement at the visit of the second week, rated at the follow-up visit (visit 3).

### Results

| Parameter | Visit 1 | Visit 2 |
|---|---|---|
| Total PASI (m±SD) Range | 8.0 ± 3.1 min 5 - max 14 | |
| TSS (m±SD) | 7.9 ± 1.7 | 4.7 ± 1.7 |
| Induration | 2.7 ± 0.4 | 1.3 ± 0.6 |
| Erythema | 2.3 ± 0.3 | 2.0 ± 0.4 |
| Scaling | 2.7 ± 0.3 | 1.4 ± 0.7 |
| ISI (m±SD) | 2.0 ± 0.6 | 1.4 ± 0.4 |
| Physician assessment | severe 2 moderate to severe 5 moderate 8 mild 6 | severe 1 moderate to severe 1 moderate 3 mild 8 almost healed 4 healed 4 |

### Clinical Conclusions

At the end of the treatment (Visit 2), 66.7% of the patients judged the treatment well acceptable and 33.3 % acceptable.

Only on three patients (14.3%) the treatment didn't provide significant results.

The treatment of patients with the composition of example 1 showed a significant improvement of TSS at Visit 2 (end of treatment). ISI has been indirectly reduced at Visit 3, i.e. after 10 days from the last administration of the composition, 17 patients on 18 (94.4%) kept the improvement showed at Visit 2 and no relapses were observed.

## Claims

1. A topical composition comprising usnic acid or a salt thereof, and a solubilizer consisting of a mixture of at least one ethoxylated hydrogenated castor oil derivative and at least one polypropylene glycol buteth.

2. The composition according to claim 1, **characterized in that** said solubilizing mixture consists of PEG-40 hydrogenated castor oil and polypropylene glycol-26-buteth-26.

3. The composition according to claim 1 or 2, **characterized in that** the weight ratio of unsalified usnic acid and said solubilizing mixture is from 1/1 to 1/2.

4. The composition according to claim 3, **characterized in that** said ratio is about 1/1.3-1.5.

5. The composition according to any one of the preceding claims, **characterized in that** it further comprises hyaluronic acid or sodium hyaluronate, both having a molecular weight from 50,000 to 300,000 Da.

6. The composition according to any one of the preceding claims, **characterized in that** it further comprises isopropyl myristate.

7. The composition according to claim 5 or 6, **characterized in that** it comprises from 0.5 to 10 wt% of usnic acid, from 0.5 to 5 wt% of sodium hyaluronate and/or from 0.5 to 10 wt% of isopropyl myristate, said amounts being expressed by weight relative to the total weight of the composition.

8. The composition according to claim 7, **characterized in that** it comprises from 1 to 5 wt% of usnic acid, from 1 to 3 wt% of sodium hyaluronate and/or from 1 to 5 wt% of isopropyl myristate, said amounts being expressed by weight relative to the total weight of the composition.

9. The composition according to any one of the preceding claims, **characterized in that** it comprises about 3 wt% of usnic acid and about 4 wt% of said solubilizing mixture, said amounts being expressed by weight relative to the total weight of the composition.

10. The composition according to any one of claims 5 to 9, **characterized in that** it comprises about 3 wt% of usnic acid, about 4 wt% of said solubilizing mixture, about 1.25 wt% of sodium hyaluronate and/or about 3 wt% of isopropyl myristate, said amounts being expressed by weight relative to the total weight of the composition.

11. The composition according to any one of the preceding claims, **characterized in that** it further comprises an anti-fungal agent.

12. The composition according to claim 11, **characterized in that** said anti-fungal agent is ketoconazole.

13. The composition of any one of claims 1 to 10, for use thereof in the treatment of skin diseases, including eczemas, dermatitis, skin wounds of various origin, sores, burns, psoriasis, proliferative keratosis and diseases caused by microbial and fungal agents.

14. The composition according to claim 11 or 12, for the use thereof in the treatment of dermatitis.

15. Use of the solubilizing mixture as defined in claims 1 and 2, to solubilize and stabilize usnic acid in water-based topical compositions.

## Patentansprüche

1. Eine topische Zusammensetzung, umfassend Usninsäure oder ein Salz davon, und einen Lösungsvermittler, der aus einer Mischung besteht, die mindestens ein ethoxyliertes hydriertes Rizinusölderivat und mindestens ein Polypropylenglykolbuteth umfasst.

2. Die Zusammensetzung gemäß Anspruch 1, **dadurch gekennzeichnet, dass** das Solubilisierungsgemisch aus PEG-40 hydriertem Rizinusöl und Polypropylenglykol-26-buteth-26 besteht.

3. Die Zusammensetzung gemäß Anspruch 1 oder 2, **dadurch gekennzeichnet, dass** das Gewichtsverhältnis von nicht salzhaltiger Usninsäure und der Solubilisierungsmischung 1/1 bis 1/2 beträgt.

4. Die Zusammensetzung gemäß Anspruch 3, **dadurch gekennzeichnet, dass** das Verhältnis etwa 1/1,3-1,5 beträgt.

5. Die Zusammensetzung gemäß einem der vorhergehenden Ansprüche, **dadurch gekennzeichnet, dass** sie ferner Hyaluronsäure oder Natriumhyaluronat umfasst, die beide ein Molekulargewicht von 50.000 bis 300.000 Da aufweisen.

6. Die Zusammensetzung gemäß einem der vorhergehenden Ansprüche, **dadurch gekennzeichnet, dass** sie ferner Isopropylmyristat umfasst.

7. Zusammensetzung gemäß Anspruch 5 oder 6, **dadurch gekennzeichnet, dass** sie 0,5 bis 10 Gew.-% Usninsäure, 0,5 bis 5 Gew.-% Natriumhyaluronat und/oder 0,5 bis 10 Gew.-% Isopropylmyristat umfasst, wobei die angegebenen Mengen als Gewicht relativ zum Gesamtgewicht der Zusammensetzung ausgedrückt werden.

8. Die Zusammensetzung gemäß Anspruch 7, **dadurch gekennzeichnet, dass** sie 1 bis 5 Gew.-% Usninsäure, 1 bis 3 Gew.-% Natriumhyaluronat und/oder 1 bis 5 Gew.-% Isopropylmyristat umfasst, wobei die angegebenen Mengen als Gewicht relativ zum Gesamtgewicht der Zusammensetzung ausgedrückt werden.

9. Die Zusammensetzung gemäß einem der vorhergehenden Ansprüche, **dadurch gekennzeichnet, dass** sie etwa 3 Gew.-% Usninsäure und etwa 4 Gew.-% der Solubilisierungsmischung umfasst, wobei die angegebenen Mengen als Gewicht relativ zum Gesamtgewicht der Zusammensetzung ausgedrückt werden.

10. Die Zusammensetzung gemäß einem der Ansprüche 5 bis 9, **dadurch gekennzeichnet, dass** sie etwa 3 Gew.-% Usninsäure, etwa 4 Gew.-% der Solubilisierungsmischung, etwa 1,25 Gew.-% Natriumhyaluronat und/oder etwa 3 Gew.-% Isopropylmyristat umfasst, wobei die angegebenen Mengen als Gewicht relativ zum Gesamtgewicht der Zusammensetzung ausgedrückt werden.

11. Die Zusammensetzung gemäß einem der vorhergehenden Ansprüche, **dadurch gekennzeichnet, dass** sie ferner ein Antimykotikum umfasst.

12. Die Zusammensetzung gemäß Anspruch 11, **dadurch gekennzeichnet, dass** das Antimykotikum Ketoconazol ist.

13. Die Zusammensetzung gemäß einem der Ansprüche 1 bis 10 zur Verwendung bei der Behandlung von Hautkrankheiten, einschließlich Ekzemen, Dermatitis, Hautwunden verschiedener Herkunft, Wunden, Verbrennungen, Psoriasis, proliferativer Keratose und Krankheiten, die durch mikrobielle und von Pilzen stammenden Stoffen verursacht werden.

14. Die Zusammensetzung gemäß Anspruch 11 oder 12 zur Verwendung bei der Behandlung von Dermatitis.

15. Verwendung der Solubilisierungsmischung gemäß den Ansprüchen 1 und 2 zur Solubilisierung und Stabilisierung von Usninsäure in topischen Zusammensetzungen auf Wasserbasis.

## Revendications

1. Composition topique comprenant de l'acide usnique ou un sel de celui-ci, et un agent solubilisant constitué d'un mélange d'au moins un dérivé d'huile de ricin hydrogénée éthoxylée et d'au moins un propylène glycol butéth.

2. Composition selon la revendication 1, **caractérisée en ce que** ledit mélange solubilisant est constitué d'huile de ricin hydrogénée PEG-40 et de propylène glycol-26-butéth-26.

3. Composition selon la revendication 1 ou 2, **caractérisée en ce que** le rapport de poids de l'acide usnique non salifié audit mélange solubilisant va de 1/1 à 1/2.

4. Composition selon la revendication 3, **caractérisée en ce que** ledit rapport est d'environ 1/1,3-1,5.

5. Composition selon l'une quelconque des revendications précédentes, **caractérisée en ce qu'**elle comprend en outre de l'acide hyaluronique ou de l'hyaluronate de sodium, tous deux ayant un poids moléculaire de 50 000 à 300 000 Da.

6. Composition selon l'une quelconque des revendications précédentes, **caractérisée en ce qu'**elle comprend en outre du myristate d'isopropyle.

7. Composition selon la revendication 5 ou 6, **caractérisée en ce qu'**elle comprend de 0,5 à 10 % en poids d'acide usnique, de 0,5 à 5 % en poids d'hyaluronate de sodium et/ou de 0,5 à 10 % en poids de myristate d'isopropyle, lesdites quantités étant exprimées en poids par rapport au poids total de la composition.

8. Composition selon la revendication 7, **caractérisée en ce qu'**elle comprend de 1 à 5 % en poids d'acide usnique, de 1 à 3 % en poids d'hyaluronate de sodium et/ou de 1 à 5 % en poids de myristate d'isopropyle, lesdites quantités étant exprimées en poids par rapport au poids total de la composition.

9. Composition selon l'une quelconque des revendications précédentes, **caractérisée en ce qu'**elle comprend environ 3 % en poids d'acide usnique et environ 4 % en poids dudit mélange solubilisant, lesdites quantités étant exprimées en poids par rapport au poids total de la composition.

10. Composition selon l'une quelconque des revendications 5 à 9, **caractérisée en ce qu'**elle comprend environ 3 % en poids d'acide usnique, environ 4% en poids dudit mélange solubilisant, environ 1,25% en poids d'hyaluronate de sodium et/ou environ 3 % en poids de myristate d'isopropyle, lesdites quantités étant exprimées en poids par rapport au poids total de la composition.

11. Composition selon l'une quelconque des revendications précédentes, **caractérisée en ce qu'**elle comprend en outre un agent antifongique.

12. Composition selon la revendication 11, **caractérisée en ce que** ledit agent antifongique est du kétoconazole.

13. Composition selon l'une quelconque des revendications 1 à 10, pour utilisation de celle-ci dans le traitement de maladies de la peau, incluant l'eczéma, la dermatite, les lésions cutanées de diverses origines, les plaies, les brûlures, le psoriasis, la kératose proliférative et des maladies causées par des agents microbiens et fongiques.

14. Composition selon la revendication 11 ou 12, pour utilisation de celle-ci dans le traitement de la dermatite.

15. Utilisation du mélange solubilisant selon les revendications 1 et 2, pour solubiliser et stabiliser l'acide usnique dans des compositions topiques à base d'eau.
